# EUROPÄISCHE PATENTSCHRIFT

(11) **EP 2 438 861 B1**
(45) Veröffentlichungstag und Bekanntmachung des Hinweises auf die Patenterteilung: **06.06.2018**
(21) Anmeldenummer: 11183754.8
(22) Anmeldetag: 04.10.2011
(51) Int. Cl.: A61B 5/1473, A61B 5/145, B01D 69/14, B01D 71/06, F16K 99/00, F15C 1/00

(54) **Medizinisches Sensorsystem zum Nachweis von zumindest einem Merkmal in zumindest einem tierischen und/oder menschlichen Körper**
Medical Sensor System for Detecting at Least One Feature in At Least One Animal Body and/or Human Body
Système de capteur médical pour contrôler au moins une caractéristique dans au moins un corps animal et/ou humain

(30) Priorität: 07.10.2010 US 390621 P
(43) Veröffentlichungstag der Anmeldung: 11.04.2012
(73) Patentinhaber: BIOTRONIK SE & Co. KG, 12359 Berlin (DE)
(72) Erfinder: Bode, Sven, 10829 Berlin (DE); Bunge, Andreas, 04105 Leipzig (DE); Biela, Sarah, 12053 Berlin (DE); Trieu, Hoc Khiem, 21394 Westergellersen (DE)
(74) Vertreter: Galander, Marcus

(56) Entgegenhaltungen:
- WO-A1-01/26708
- WO-A1-02/20877
- DE-A1- 19 853 286
- DE-A1-102006 025 344
- US-A- 5 290 240
- US-A1- 2004 006 300
- US-A1- 2006 004 272
- US-A1- 2008 115 361
- US-A1- 2008 213 133
- US-A1- 2009 024 114
- US-A1- 2009 317 445

## Beschreibung

Die Erfindung betrifft ein medizinisches Sensorsystem zum Nachweis von zumindest einem Merkmal in zumindest einem tierischen und/oder menschlichen Körper nach dem Oberbegriff des Patentanspruchs 1 und ein Verfahren gemäß dem Oberbegriff des Patentanspruchs 13.

In der Medizin kommen Sensorsysteme zum Einsatz, bei denen Teile des Systems direkt in einen Körper eines Patienten eingesetzt bzw. implantiert werden, um möglichst genau und unmittelbar physiologische Ist-Zustände zu erfassen.

Aus der WO 2008/154416 A2 in Verbindung mit US 6,527,762 B1 ist beispielsweise ein in den Körper implantierbarer Sensor bekannt bei dem ein Verschluss eines Reservoirs des Sensors von einer dünnen Metallfolie gebildet wird, die durch eine angelegte Spannung an gewissen Stellen irreversibel thermisch entfernt wird wonach das Innere des Sensors freiliegt und zu jeder Zeit Degradationsprozessen unterliegt. Hierbei können Reste der Metallfolie in den Körper gelangen, die schädlich für den Körper sein können. Die Dokumente US2006004272 und US2008115361 offenbaren den Oberbegriff der Ansprüche 1 und 13.

Der Erfindung liegt die Aufgabe zugrunde, ein medizinisches Sensorsystem bereitzustellen, das flexibel einsetzbar und robust bzw. fehlerunanfällig ausführbar ist.

Die Aufgabe wird erfindungsgemäß durch die Merkmale der Patentansprüche 1 und 13 gelöst. Günstige Ausgestaltungen und Vorteile der Erfindung ergeben sich aus den weiteren Ansprüchen, der Zeichnung und der Beschreibung.

Die Erfindung geht aus von einem medizinischen Sensorsystem zum Nachweis von zumindest einem Merkmal in zumindest einem tierischen und/oder menschlichen Körper mit zumindest einem Sensor und zumindest einem Verschluss eines Reservoirs des Sensors, aufweisend zumindest ein Reservoir mit zumindest einem Nachweissystem für eine Merkmalsmessung des Merkmals, und zumindest einem Verschluss des Reservoirs des Sensors, wobei der Verschluss als steuerbare, organische Membran ausgebildet ist, welche zumindest eine Pore aufweist, deren Durchmesser reversibel veränderbar ist, wobei für einen ersten Fall in einem ersten Schritt einer Referenzmessung ein erster Durchmesser der Pore der steuerbaren, organischen Membran so einstellbar ist, dass das Merkmal nicht in ein Probenvolumen des Reservoirs eindringen kann und alle Moleküle, die kleiner als das Merkmal sind, in das Probenvolumen eindringen können, wobei bei der Referenzmessung mit dem Nachweissystem Störsignale als ein Messergebnis der Referenzmessung ermittelbar sind und in einem zweiten Schritt einer Merkmalsmessung ein zweiter Durchmesser der Pore der steuerbaren, organischen Membran so einstellbar ist, dass das Merkmal in das Probenvolumen eindringen kann, wobei bei der Merkmalsmessung mit dem Nachweissystem das Merkmal als ein Messergebnis der Merkmalsmessung messbar ist und das Messergebnis der Referenzmessung als Hintergrundsignal von dem Messergebnis der Merkmalsmessung abgezogen wird, wodurch ein Endmesswert ermittelbar ist und für einen zweiten Fall bei einer Referenzmessung ein erster Durchmesser der Pore der steuerbaren, organischen Membran eines zweiten Sensors so einstellbar ist, dass das Merkmal nicht in ein Referenzvolumen des zweiten Sensors eindringen kann und alle Moleküle, die kleiner als das Merkmal sind, in das Referenzvolumen eindringen können, wobei bei der Referenzmessung mit dem Nachweissystem Störsignale als ein Messergebnis der Referenzmessung ermittelbar sind und bei einer Merkmalsmessung ein zweiter Durchmesser der Pore der steuerbaren, organischen Membran des ersten Sensors so einstellbar ist, dass das Merkmal in ein Probenvolumen des Reservoirs eindringen kann, wobei bei der Merkmalsmessung mit dem Nachweissystem das Merkmal als ein Messergebnis der Merkmalsmessung bestimmbar ist und das Messergebnis der Referenzmessung als Hintergrundsignal von dem Messergebnis der Merkmalsmessung abgezogen wird, wourch ein Endmesswert ermittelbar ist. Durch die erfindungsgemäße Ausgestaltung kann ein Sensorsystem bereitgestellt werden, dass flexibel an sein Anwendungsgebiet angepasst werden kann. Zudem kann es besonders schonend für ein Einsatzgebiet wie dem tierischen und/oder menschlichen Körper biokompatibel ausgestaltet werden.

In diesem Zusammenhang soll unter einem "Sensorsystem" insbesondere ein System mit zumindest einem Sensor verstanden werden. Ferner kann das Sensorsystem weitere Bestandteile, wie weitere Sensoren, ein Gehäuse, Elektronikkomponenten, eine Energieversorgung, eine Telemetrieeinheit, eine Steuereinheit, eine Verankerung und/oder jedes andere, dem Fachmann für einsetzbar erscheinendes Bestandteil, aufweisen. Unter einem "Merkmal" soll insbesondere ein Parameter, wie ein eine Menge eines Stoffs und/oder eines Analyten, wie ein Fett, ein Salz, ein Ion, ein Polyelektrolyt, ein Zucker, ein Nucleotid, DNA, RNA, ein Peptid, ein Protein, ein Antikörper, ein Antigen, ein Medikament, ein Toxin, ein Hormon, ein Neurotransmitter, ein Metabolit, ein Stoffwechselprodukt, der Wassergehalt, der Hämatokritwert und/oder jeder andere, dem Fachmann für zweckdienlich erscheinendes Analyt verstanden werden. Unter einem "Merkmal" sind auch so genannte Biomarker zu verstehen, die einen variablen Bestandteil des menschlichen oder tierischen Körpers bilden, wie beispielsweise Albumine/Globuline, Alkalische Phosphatase, Alpha-1-Globulin, Alpha-2-Globulin, Alpha-1-Antitrypsin, Alpha-1-Fetoprotein, Alpha-Amylasen, Alpha-Hydroxybutyrat-Dehydrogenase, Ammoniak, Antithrombin III, Bicarbonat, Bilirubin, Carbohydrate-Antigen 19-9, Carcino-embryonale Antigene, Chlorid, Cholesterin, Cholinesterase, Cobalamin/Vitamin B12, Coeruloplasmin, C-reaktive Proteine, Cystatin C, D-Dimere, Eisen, Erythropoetin, Erythrozyten, Ferritin, Fetuin A Fibrinogen, Folsäure/Vitamin B9, Freie Tetrajodthyronine (fT4), Freie Trijodthyronine (fT3), Gamma-Glutamyltransferase, Glukose, Glutamat-Dehydrogenase, Glutamat-Oxalazetat-Transaminase, Glutamat-Pyruvat-Transaminase, Glykohämoglobin, Hämatokrit, Hämoglobin, Haptoglobin, Harnsäure, Harnstoff, HDL-Cholesterin, Homozystein, Immunglobulin A, Immunglobulin E, Immunglobulin G, Immunglobulin M, INR, Kalium, Kalzium, Kreatinin, Kreatin-Kinase, Kupfer, Laktat, Laktat-Dehydrogenase, LDL-Cholesterin, Leukozyten, Lipase, Lipoprotein, Magnesium, korpusukuläre Hämoglobine, Myoglobin,, Natrium, NT-proBNP/BNP, Phosphat, Prostataspezifische Antigene, Retikulozyten, Thrombozyten, Transferrin, Triglyzeride, Troponin T oder auch Medikamente wie Muskarinrezeptor-Antagonisten, neuromuskulär blockierende Stoffe, Cholesterinesterase-Hemmstoffe, Adrenozeptor-Agonisten, indirekt wirkende Sympathomimetika, Methylxanthine, alpha-Adrenorezeptor-Antagonisten, Mutterkornalkaloide, beta-Adrenozeptor-Antagonisten, Inaktivierungshemmstoffe, Antisympathonika, 5-HT- Rezeptor-agonisten, Histaminrezeptor-Agonisten, Hiastaminrezeptor-Antagonisten, Analgetika, Lokalanästhetika, Sedativa, Antikonvulsiva, Konvulsiva, Muskelrelaxantien, Antiparkinsonmittel, Neuroleptika, Antidepressiva, Lithium, Tranquillantien, Immunsuppressiva, Antirheumatika, Antiarrhythmika, Antibiotika, ACE-Hemmer, Aldosteronrezeptor-antagonisten, Diuretika, Vasodilatatoren, positiv inotrope Substanzen, antithrombotische/thrombolytische Substanzen,Laxantien, Antidiarrhoioka, Pharmake bei Adipositas, Uricostatika, Uricosurika, Lipidsenker, Antidiabetika, Anithypoglykämia, Hormone, Iodsalze, Threostatika, Eisen, Vitamine, Spurenelemente, Virostatika, Antimykotika, Antituberkulotika und Substanzen zur Tumorchemotherapie Generell wäre jedoch auch jedes andere, dem Fachmann als zweckdienlich erscheinendes Merkmal denkbar. Bevorzugt bezieht sich das Merkmal auf einen variablen Bestandteil des tierischen und/oder menschlichen Körpers. Vorteilhafterweise dient das Sensorsystem zum Nachweis eines Mitglieds der Cystatin-Familie der Cysteinprotease-Inhibitoren und insbesondere zum Nachweis von Cystatin C und ist somit ein Cystatin-C-Sensor.

Des Weiteren soll unter einem Sensor insbesondere ein Bauteil verstanden werden, das eine physikalische und/oder chemische Eigenschaft des Parameters in einer Umgebung des Sensors qualitativ und/oder quantitativ etwa als Messgröße erfassen kann. Unter einem "Reservoirs eines Sensors" bzw. einem "Sensorreservoirs" soll hier insbesondere ein Raum, eine Kammer und/oder eine Kavität des Sensors verstanden werden, mit dem ein Nachweissystem des Sensors in Kontakt steht und/oder an und bevorzugt in der das Nachweissystem angeordnet ist. Ferner schließt das Sensorreservoir zumindest ein Volumen ein und insbesondere ein Probenvolumen, das das nachzuweisende Merkmal enthält bzw. aufweist. Ein "Verschluss" stellt hier insbesondere eine Vorrichtung und/oder ein Bestandteil des Sensorreservoirs dar, die bzw. der das Sensorreservoir in zumindest einem Betriebszustand des Sensorsystems verschließt bzw. ein Eindringen und/oder ein Austreten des Probenvolumens in/aus dam Sensorreservoir verhindert. Somit stellt der Verschluss bzw. die steuerbare, organische Membran einen funktionalen Bestandteil des Sensors dar. Ferner soll unter "steuerbar" insbesondere verstanden werden, dass die Membran mittels zumindest eines Signals von zumindest einem ausgewählten Anfangszustand in zumindest einen ausgewählten Endzustand überführt werden kann. Vorteilhaft ist das Signal ein von außerhalb des Sensorsystems einwirkender Einfluss, wie beispielsweise Strahlung, Infrarot, Ultraschall, ein elektrisches Feld, ein magnetisches Feld, sichtbares Licht, ein Protein, ein Peptid, ein Polyelektrolyt, eine pH-Wertänderung, eine Veränderung der Ionenkonzentration, eine Temperaturänderung und/oder jede andere, dem Fachmann für sinnvoll erscheinende Einwirkung. Bevorzugt ist ein Volumen der Membran steuerbar. In diesem Zusammenhang soll unter dem Begriff "organische Membran" insbesondere eine Trennschicht und/oder ein dünner Film verstanden werden, die/der zumindest einen Bestandteil aufweist, der auf einer Kohlenstoffverbindung basiert.

Vorteilhafterweise kann die steuerbare, organische Membran in Abhängigkeit von dem Auslösesignal ihren Zustand so ändern, dass zumindest ein Teil der Membran durchlässig für den Analyten ist, wodurch der Analyt einen guten Zugang zum Nachweissystem erhält.

Des Weiteren wird vorgeschlagen, dass die steuerbare, organische Membran zwischen einem geöffneten und einem geschlossenen Zustand des Reservoirs stufenlos reversibel veränderbar ist. Unter "stufenlos" soll hier insbesondere die Möglichkeit verstanden werden, die Öffnungsweite der Membran bis zu einer maximalen Grenze beliebig weit einzustellen. Unter "reversibel" soll hier insbesondere umkehrbar verstanden werden. Durch die Realisierung der reversiblen Änderung kann das sich im Sensorreservoir befindliche Nachweissystem vorteilhaft vor interferierenden Molekülen geschützt werden, die den Sensor angreifen, degradieren und/oder zerstören könnten. Auch Störfaktoren, die eine einwandfreie Funktion des Sensors beinträchtigen können, werden so vorteilhaft minimiert. Dadurch kann ein besonders langlebiges Sensorsystem bereitgestellt werden.

Ferner ist es zweckmäßig, wenn die steuerbare, organische Membran vor einem ersten Einsatz bzw. einem ersten Messbetrieb des Sensors geschlossen ist, wodurch der Sensor vor seiner ersten Inbetriebnahme effektiv vor störenden Einflüssen, wie Schmutz, Staub, zu hohe Feuchtigkeit, Trockenheit, Temperaturschwankungen und /oder schädlichen Molekülen geschützt ist. Des Weiteren kann es vorteilhaft sein, wenn die steuerbare, organische Membran zwischen den einzelnen Messungen verschließbar ist, wodurch das Sensorsystem Komponenten stabil realisiert werden kann.

Dadurch, dass die steuerbare, organische Membran zumindest eine Pore aufweist, deren Durchmesser reversibel veränderbar ist, kann ein Zustand der Membran und ein Übertritt des Analyten in das Probevolumen konstruktiv besonders einfach flexibel, insbesondere für unterschiedliche Moleküle und/oder Analyten, gestaltet werden kann. Ferner sind zumindest ein Öffnen und/oder ein Schließen der Pore der Membran steuerbar. Die Pore ist bevorzugt eine Nanopore und kann einen maximalen Durchmesser von 1 µm annehmen. Grundsätzlich kann die Pore auch ein von der runden Form abweichende Kontur, wie dreieckig, rechteckig, sternförmig, oval und/oder jede andere, dem Fachmann für zweckdienlich erscheinende Kontur aufweisen. Durch die Nanopore kann einfach verhindert werden, dass Strukturen, wie beispielsweise Zellen, große Moleküle oder Molekülaggregate, die eine größere Erstreckung wie der Durchmesser der Nanopore haben, aus der Sensorumgebung in das Probenvolumen eindringen, um dort das Nachweissystem zu stören. Vorteilhaft weist die Membran eine Vielzahl von gleichartigen Poren auf, die gleichmäßig über eine Oberfläche der Membran verteilt angeordnet sind. Grundsätzlich wäre auch eine inhomogene Verteilung der Poren denkbar. Vorteilhafterweise ist der Porendurchmesser stufenlos einstellbar, was eine Anwendung mit einer Vielzahl an Analyten ermöglicht.

Ferner ist es vorteilhaft, wenn die steuerbare, organische Membran zumindest ein Material aufweist, dessen Redoxzustand änderbar ist. Hierbei kann der Redoxzustand chemisch, elektrisch und/oder auf jeder anderen, dem Fachmann für anwendbar erscheinende Weise änderbar sein. Durch die erfindungsgemäße Ausgestaltung kann die Durchlässigkeit der Membran konstruktiv einfach und zuverlässig verändert werden.

Ein besonders einfacher und komfortabler Sensor kann bereitgestellt werden, wenn die steuerbare, organische Membran elektrisch steuerbar ist. Hierbei ist insbesondere der Porendurchmesser der Pore bzw. der Poren der Membran elektrisch steuerbar. Dies erfolgt vorteilhafterweise durch Anlegen einer Spannung von maximal ca. 2 V. Bei vollständig geöffneten Nanoporen hat das seinen Redoxzustand ändern könnende Material sein minimales Volumen. Das Anlegen einer Spannung führt zu einer Volumenvergrößerung des Materials, wodurch sich der freie Durchmesser verkleinert. Die Volumenvergrößerung ist entweder abhängig von der Höhe der Spannung wenn diese eine bestimmte konstante Zeitspanne anliegt oder sie ist von der Zeitspanne abhängig, während derer eine konstante Spannung anliegt. In beiden Fällen muss die Spannung nur für eine bestimmte Zeitdauer angelegt werden. Die Reversion des Vorganges erfolgt analog durch Anlegen einer Spannung umgekehrter Polarität, die dann wieder zu einer Volumenreduktion des Materials führt. Die Volumenänderung ist ebenfalls von dem Strukturdesign des Materials abhängig.

Bevorzugt ist das Material, das seinen Redoxzustand ändern kann, ein elektroaktives Material bzw. ein elektroreaktives Polymer. Wird als elektroaktives Polymer z. B. eine Mischung aus Polypyrrol (PPy) und Dodecylbenzolsulfonsäure (DBS) eingesetzt, werden während einer spannungsgesteuerten Reduktion des Polymers Natriumionen in das Polymer eingelagert. Diese Einlagerung von Natriumionen erzeugt eine stark laterale Volumenänderung des elektroaktiven Polymers, das damit die Poren für den Analyten verschließt. Die Reversibilität dieses Vorganges ermöglicht eine kontrollierte Öffnung und ein kontrolliertes Schließen der Poren und damit eine kontrollierte, wiederholbare Messung mittels des Sensors. Über den Grad der Reduktion des Polymers ist eine partielle Volumenänderung des Polymers möglich. Die Redoxzustände des elektroaktiven Polymers werden über unterschiedliche angelegte Spannungen erzeugt und bei einer Abschaltung der Spannung beibehalten. Dadurch kann das Polymer und damit der Porendurchmesser vorteilhaft auf verschieden große Analyten eingestellt werden.

Ferner wird vorgeschlagen, dass das zumindest ein Nachweissystem, mit dem das zu messende Merkmal nachgewiesen werden kann, eines bekannter Art ist. Dieses Nachweissystem ist dadurch gekennzeichnet, dass es eine Rezeptorschicht aufweist, die eine messbare Reaktion mit dem zu messenden Merkmal bewirkt, wodurch ein zuverlässiges und vielfach erprobtes Sensorsystem zum Einsatz kommt. Diese Rezeptorschicht stellt vorteilhafterweise zumindest einen Teil des Nachweissystems dar. Ein "Rezeptor" kann hierbei aus der Stoffklasse der Peptide, Proteine - insbesondere der Enzyme, Antikörper und deren Fragmente, RNA, DNA, Nucleotide, Fette, Zucker, Salze, Ionen, zyklische Makromoleküle, wie Ionophore und Kronenether sowie Kryptanden, nichtzyklische Makromoleküle und/oder jeder anderen, dem Fachmann für sinnvoll erscheinenden Stoffklasse sein. Die Rezeptorschicht ist bevorzugt eine Antikörperschicht auf einem seFET.

In einer weiteren Ausgestaltung der Erfindung ist vorgesehen, dass die Membran bzw. das Redoxzustand verändernde Material bzw. das Polymer auf eine nanoporöse Substanz als Trägerstruktur aufgebracht ist, wodurch eine besonders gute Funktionalität erreicht werden kann. Bevorzugt ist die Membran bzw. das Redoxzustand verändernde Material bzw. das Polymer zumindest an einer Innenoberfläche von Poren der nanoporösen Substanz bzw. Trägerstruktur angeordnet. Hierdurch können die Nanoporen der Trägerstruktur vorteilhaft als Grundgerüst der Porenstruktur verwendet werden. Der Porendurchmesser ist dabei abhängig vom nachzuweisenden Analyten. Bevorzugt wird der Porendurchmesser so gewählt, dass die Membran für Moleküle des Analyten durchlässig ist, jedoch für größere Moleküle eine Barriere darstellt. Grundsätzlich können, falls es sich beim nachzuweisenden Analyten um ein Protein oder ein ähnlich großen Analyten handelt die Poren einen maximalen Durchmesser von 1 µm, bevorzugt von maximal 250 nm, weiterhin bevorzugt von 100 nm, vorteilhafterweise von maximal 50 nm und besonders bevorzugt von maximal 10 nm aufweisen. Bei kleineren Analyten können die Poren einen maximalen Durchmesser von 500 nm, bevorzugt von maximal 100 nm, weiterhin bevorzugt von 50 nm, vorteilhafterweise von maximal 10 nm und besonders bevorzugt von maximal 1 nm aufweisen. Generell kann die Pore auch eine beliebig andere Kontur, wie eine dreieckige, rechteckige, sternförmige, ovale und/oder jede andere, dem Fachmann für zweckdienlich erscheinende Kontur aufweisen, wobei die maximale Erstreckung die oben genannte Staffelung aufweist.

Die nanoporöse Substanz weist bevorzugt ein Metalloxid wie Al₂O₃, In₂O₃, MgO, ZnO, CeO₂ Co₃O₄ auf und/oder weist die Trägerstruktur für die steuerbare, organische Membran bzw. das Polymer besonders bevorzugt zumindest TiO₂ auf. Grundsätzlich wäre jedoch auch jede andere nanoporöse Substanz einsetzbar. Mittels der Verwendung von TiO₂ kann eine Trägerstruktur vorteilhaft eingesetzt werden, die besonders gewichtsarm, biokompatibel und bioinert ist. Zudem ist die Nanoporenstruktur, aufgebaut aus Nanoröhrchen, sehr einfach und reproduzierbar zu synthetisieren. Diese sehr regelmäßigen Strukturen können relativ einfach durch einen Anodisierungsprozess erzeugt werden. Die Porengröße und Schichtdicke dieses Substrats können durch Parameter während der Erzeugung leicht eingestellt werden. In der Regel ist die Schichtdicke mit mehreren hundert Mikrometern deutlich größer als der Durchmesser der Nanoröhrchen.

Bevorzugt ist das komplette Sensorsystem biologisch abbaubar, wodurch es nach seinem Funktionsverlust Patienten schonend nicht wieder aus dem Körper entfernt werden muss. Zudem kann verhindert werden, dass schädliche Stoffe, wie beispielsweise bei einem Einsatz eines nicht biokompatiblen und abbaubaren Sensors gesundheitsgefährdend in den Körper gelangen.

Besonders vorteilhaft ist die steuerbare, organische Membran auf eine Trägerstruktur aufgebracht ist, die eine hohe Biokompatibitität aufweist, wodurch vorteilhaft Abstoßungs- und Entzündungsreaktionen gesundheitsfördernd minimiert und bevorzugt unterbunden werden können.

Der Sensor ist vorteilhaft zur quantitativen Bestimmung des Merkmals ausgebildet, wobei so eine Konzentrationsbestimmung des Analyten, beispielsweise in Körperflüssigkeiten, wie Blut, Urin, interstitieller Flüssigkeit oder Tränenflüssigkeit, einfach bestimmt werden kann.

Da derselbe Bereich als ein erster Sensor und als ein zweiter Sensor vorgesehen ist, kann die quantitative Bestzimmung besonders Bauteil und Baurraum sparend erfolgen. Hierbei ist der erste Sensor als ein Messsensor und der zweite Sensor als ein Referenzsensor ausgebildet. Der Messsensor und der Referenzsensor sind einstückig miteinander ausgebildet, wobei hier unter "einstückig" insbesondere verstanden werden soll, dass Messsensor und der Referenzsensor von demselben Bauteil gebildet sind und/oder nur unter Funktionsverlust voneinander getrennt werden können. Das Sensorreservoir ist so ausgebildet, das es in einem ersten Modus zur Referenzmessung und in einem zweiten, zeitlich dem ersten Modus nachfolgenden Modus zur Analytmessung dient. Hierbei wird eine Porengröße bei der Referenzmessung so eingestellt, dass sie kleiner als ein Durchmesser des Analyten ist, sodass dieser nicht in das Probevolumen eindringen kann. Moleküle oder Strukturen, die kleiner als der Analyt sind, können hingegen in das Probenvolumen eindringen. Bei der Analytmessung wird die Porengröße nun an die Größe des Analyten angepasst, wodurch dieser ins Probenvolumen eindringen kann. Das Messsignal der Referenzmessung wird nun als Hintergrundsignal von dem Messsignal der Analytmessung abgezogen, wodurch ein Endmesswert ermittelt wird. So kann das Hintergrundsignal von Störeffekten konstruktiv einfach bestimmt werden.

Bei der alternativen Ausgestaltung des Sensorsystems sind zwei unterschiedliche Bereiche als ein erster Sensor und als ein zweiter Sensor vorgesehen, wodurch zeitsparend die Analyt- und Referenzmessung zeitgleich erfolgen kann. Der erste und der zweite Sensor bzw. der Messsensor und der Referenzsensor sind bevorzugt räumlich voneinander getrennt im Sensorsystem angeordnet. Der zweite Sensor weist ebenso ein Referenzreservoir, das ein Referenzvolumen einschließt, und eine schaltbare, organische Membran und insbesondere eine elektrisch schaltbare Membran auf. Die beiden Sensoren unterscheiden sich durch eine Konfiguration ihrer schaltbaren Membranen und insbesondere durch die daran jeweils eingestellte Porengröße. Die eingestellte Porengröße der steuerbaren, organischen Membran des ersten Bereichs bzw. des ersten Sensors/Messsensors lässt den Analyten in das Probenvolumen. Eine kleiner eingestellte Porengröße der steuerbaren, organischen Membran des zweiten Bereichs, bzw. des zweiten Sensors/Referenzsensor hält den Analyten ab, in das Referenzvolumen zu gelangen. Durch Korrigieren des gemessenen Signals des Messsensors mittels des Referenzsignals erhält man den Endmesswert. Die unterschiedlichen Porositäten für Mess- und Referenzsensor werden durch entsprechend unterschiedlich hohe Spannungen und/oder Zeitdauer der anliegenden Spannung an den zu den Sensoren gehörenden Membranen eingestellt.

Dieser Aufbau hat den Vorteil, dass sowohl Messsensor und Referenzsensor das gleiche Nachweissystem enthalten und somit ein Drift und Alterungsprozesse gleich ablaufen. Somit können bei Ermittlung des Endmesswerts mittels des Messsensors, des Referenzsensors oder beider Sensoren Aussagen über den Drift (z.B. Degradierung des Nachweissystems, Veränderung der Temperatur und andere Effekte) des Sensorsystems vorteilhaft gesammelt werden. Über einen geeigneten Korrekturterm können die nachfolgenden Messwerte bzgl. der Drift korrigiert werden, bzw. über eine geeignete Methode (elektrischer, mechanischer oder chemisch/biochemischer Natur) kompensiert werden. Ist die Drift zu groß, dass diese Mechanismen nicht mehr greifen, kann bei einem Arraysystem gleicher Sensorsysteme ein neues Sensorsystem aktiviert werden.

Zweckmäßigerweise geht die Erfindung ferner aus von einem medizinischen Sensorarray mit zumindest zwei Sensorsystemen. Hierbei kann es sich um die gleichen Sensorsysteme handeln, die zeitlich nacheinander geschaltet den gleichen Analyten nachweisen bzw. dessen Konzentration bestimmen. Weiterhin können die Sensorsysteme gleichzeitig für die Analytmessung und für die Referenzmessung benutzt werden. Alternativ ist ebenfalls ein Array von Sensorsystemen möglich, die gleichzeitig oder zeitlich nacheinander verschiedene Analyten und/oder deren Konzentration nachweisen können. Hierdurch kann vorteilhaft eine beschränkte Lebensdauer eines Sensorsystems, mittels eines Arrays von mehreren Sensorsystemen auf eine gute Gesamtlebensdauer verlängert werden.

Vorteilhaft geht die Erfindung zudem aus von einem medizinischen Implantat mit zumindest einem medizinischen Sensorsystem und/oder mit einem medizinischen Sensorarray. In diesem Zusammenhang soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantate, wie beispielsweise ein Implantat zur Aufzeichnung von physiologischen Parametern, ein Herzschrittmacher, ein Defibrillator, ein Hirnschrittmacher, ein Nierenschrittmacher, ein Zwölffingerdarm-Schrittmacher, ein Herzimplantat, künstliche Herzklappen, ein Cochleaimplantat, ein Retina Implantat, ein zahnmedizinisches Implantat, ein Implantat zum Gelenkersatz, eine Gefäßprothesen oder besonders vorteilhaft ein Stent, wie ein Koronarstent, ein Nierenaterienstent oder ein Harnleiterstent sowie ein Drug-Delivery-System. Mit so einem Implantat können insbesondere Erkrankungen, wie beispielsweise Herzinsuffizienz, Bluthochdruck, Niereninsuffizienz und/oder Diabetes Mellitus als häufige, behandlungsintensive und somit teure chronische Erkrankungen besser beobachtet und therapiert werden.

Zudem geht die Erfindung aus von einem Verfahren zum Betreiben eines medizinischen Sensorsystems zum Nachweis von zumindest einem Merkmal in zumindest einem tierischen und/oder menschlichen Körper mit zumindest einem ersten Sensor, aufweisend zumindest ein Reservoir mit zumindest einem Nachweissystem für eine Merkmalsmessung des Merkmals, und zumindest einem Verschluss des Sensorreservoirs, wobei der Verschluss von einer steuerbaren, organischen Membran gebildet wird, die reversibel zwischen einem geöffneten und einem geschlossenen Zustand des Reservoirs verändert wird und welche zumindest eine Pore aufweist, deren Durchmesser reversibel verändert wird.

Es wird vorgeschlagen, dass für einen ersten Fall in einem ersten Schritt einer Referenzmessung ein erster Durchmesser der Pore der steuerbaren, organischen Membran so eingestellt wird, dass das Merkmal nicht in ein Probenvolumen des Reservoirs eindringen kann und alle Moleküle, die kleiner als das Merkmal sind, in das Probenvolumen eindringen können, wobei bei der Referenzmessung mit dem Nachweissystem Störsignale als ein Messergebnis der Referenzmessung ermittelt werden und in einem zweiten Schritt einer Merkmalsmessung ein zweiter Durchmesser der Pore der steuerbaren, organischen Membran so eingestellt wird, dass das Merkmal in das Probenvolumen eindringen kann, wobei bei der Merkmalsmessung mit dem Nachweissystem das Merkmal als ein Messergebnis der Merkmalsmessung gemessen wird und das Messergebnis der Referenzmessung als Hintergrundsignal von dem Messergebnis der Merkmalsmessung abgezogen wird, wodurch ein Endmesswert ermittelt wird und für einen zweiten Fall bei einer Referenzmessung ein erster Durchmesser der Pore der steuerbaren, organischen Membran eines zweiten Sensors so eingestellt wird, dass das Merkmal nicht in ein Referenzvolumen des zweiten Sensors eindringen kann und alle Moleküle, die kleiner als das Merkmal sind, in das Referenzvolumen eindringen können, wobei bei der Referenzmessung mit dem Nachweissystem Störsignale als ein Messergebnis der Referenzmessung ermittelt wird und bei einer Merkmalsmessung ein zweiter Durchmesser der Pore der steuerbaren, organischen Membran des ersten Sensors so eingestellt wird, dass das Merkmal in ein Probenvolumen des Reservoirs eindringen kann, wobei bei der Merkmalsmessung mit dem Nachweissystem das Merkmal als ein Messergebnis der Merkmalsmessung bestimmt wird und das Messergebnis der Referenzmessung als Hintergrundsignal von dem Messergebnis der Merkmalsmessung abgezogen wird, wodurch ein Endmesswert ermittelt wird. Durch die erfindungsgemäße Ausgestaltung kann das sich im Sensorreservoir befindliche Nachweissystem vorteilhaft vor interferierenden Molekülen geschützt werden, die den Sensor angreifen, degradieren und/oder zerstören könnten. Auch Störfaktoren, die eine einwandfreie Funktion des Sensors beinträchtigen können, werden so vorteilhaft minimiert.

Es wird somit zum Nachweis des Merkmals in dem ersten Schritt der erste Durchmesser der Pore der steuerbaren, organischen Membran eingestellt und in dem zweiten Schritt der zweite Durchmesser der Pore eingestellt, wobei der erste Durchmesser kleiner als der zweite Durchmesser ist. Dadurch kann besonders Bauteil und Baurraum sparend mittels eines Sensorreservoirs sowohl das Merkmal bzw. der Analyt wie auch ein Hintergrundsignal ermittelt werden.

In der weiteren Ausgestaltung der Erfindung wird zum Nachweis des Merkmals an der steuerbaren, organischen Membran des zweiten Sensors der erste Durchmesser einer Pore der steuerbaren, organischen Membran eingestellt wird und an der steuerbaren, organischen Membran des ersten Sensors der zweite Durchmesser der Pore der steuerbaren, organischen Membran eingestellt, wobei der erste Durchmesser kleiner als der zweite Durchmesser ist. Hierdurch kann eine Ermittlung eines Korrekturwerts vorteilhaft Zeit und Arbeitsaufwand sparend zeitgleich zu einer Ermittlung des Analytmesswertes erfolgen.

Vorteilhaft trägt die steuerbare, organische Membran zur Unterbindung von Biofouling auf der Oberseite zumindest eine Struktur, die ein Anheften von Zellen und Molekülen unterbindet. Hierbei können TiO₂-Nanostrukturen für sich genommen bereits die Anhaftung von Zellen verhindern.

Darüber hinaus kann die Oberfläche des Implantats bzw. Teile der Oberfläche abhängig von der Anwendung hydrophobe oder hydrophile Eigenschaften besitzen, sie können sowohl einen kationischen oder anionischen bzw. metallischen Charakter tragen. An die Oberfläche können anorganische oder organische Moleküle über physikalische Adsorption oder kovalente Bindung verknüpft werden, wie z.B. Polymere, Peptide, Proteine, Aptamere, molekular geprägte Polymere, RNA, DNA, siRNA und Nanopartikel. Die Oberfläche kann eine Nano- oder Mikrostrukturierung tragen. Es können zu Strukturierung auf der Oberfläche sowohl runde, sphärische, zylindrische, kegelfärmige, quadratische, rechteckige, wie auch längliche Strukturen aufgebracht wie auch abgetragen werden. Dazu zählen Rillen, Röhren, Vollzylinder, Hohlzylinder, Kugeln, Halbkugeln, Quader und Würfel.

Es ist auch eine teilweise bioresorbierbare oder biodegradierbare Oberfläche denkbar.

Es wäre ebenfalls vorstellbar, dass auf die Oberfläche gezielt Strukturen beispielsweise aus einer Körperflüssigkeit, wie z.B. bestimmte Proteine oder Zellen angelockt, immobilisiert und die Zellen zur Proliferation gebracht werden. Hierfür können Antigene, Peptide, Proteine, Antikörper, Aptamere, molekular-geprägten Polymere und Oligonikleotide (DNA, RNA, PNA, LNA) an die Oberfläche adhäriert oder kovalent gebunden werden.

Ferner kann der Sensor bzw. das *in vivo* Messgerät eine Telemetrievorrichtung enthalten, mittels derer die gemessenen Werte an ein externes Gerät übertragen werden können. Die Telemetrievorrichtung kann bidirektional ausgeführt sein, so dass eine Steuerung des implantierten Sensors oder Messgerätes durch ein externes Gerät erfolgen kann. Ebenso kann der Sensor bzw. das *in vivo* Messgerät eine Teilkomponente eines Body-Area-Networks sein, d.h. parallel erfassen weitere ebenfalls über drahtlose Telemetrie miteinander und/oder mit einem externen Gerät kommunizierende Sensoren physiologisch relevante Parameter wie Druck, Puls, EKG, EEG, biochemische Größen und/oder andere, dem Fachmann für zweckdienlich erscheinende Parameter.

Die Erfindung ist nachfolgend beispielhaft, anhand in Zeichnungen dargestellter Ausführungsbeispiele, näher erläutert. Es zeigen:
- Fig. 1: eine schematische Ansicht eines erfindungsgemäßen Sensorsystems von oben,
- Fig. 2a: einen Schnitt entlang der Linie II-II durch das Sensorsystem der Fig. 1 mit geschlossenen Poren in einer schematischen Darstellung,
- Fig. 2b: das Sensorsystem der Fig. 2a in einem geöffneten Zustand der Poren für eine Referenzmessung,
- Fig.2c: das Sensorsystem der Fig. 2a in einem geöffneten Zustand der Poren für eine Analytmessung,
- Fig. 3a: eine Detaildarstellung einer Pore aus Fig. 2a im geschlossenen Zustand,
- Fig. 3b: eine Detaildarstellung einer Pore aus Fig. 2c im geöffneten Zustand,
- Fig. 4: das Sensorsystem der Fig. 1 mit weiteren Komponenten,
- Fig. 5a: ein Implantat ausgestattet mit einem Sensorsystem gemäß der Fig. 1
- Fig. 5b: ein alternatives Implantat ausgestattet mit einem Sensorarray mit vier Sensorsystem gemäß der Fig. 1,
- Fig. 6: einen Schnitt durch ein alternatives Sensorsystem mit einem Messsensor und einem Referenzsensor mit unterschiedlich weit geöffneten Poren in einer schematischen Darstellung,
- Fig. 7a: ein weiteres alternatives Implantat ausgestattet mit einem Sensorsystem gemäß der Fig. 6 und,
- Fig. 7b: ein viertes Implantat ausgestattet mit einem Sensorarray mit vier Sensor-system gemäß der Fig. 6.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verwiesen.

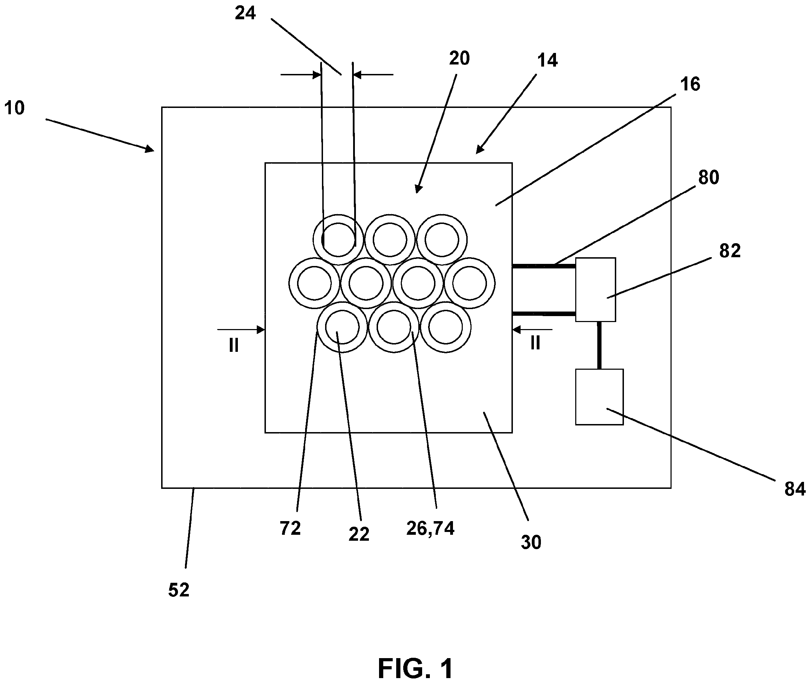

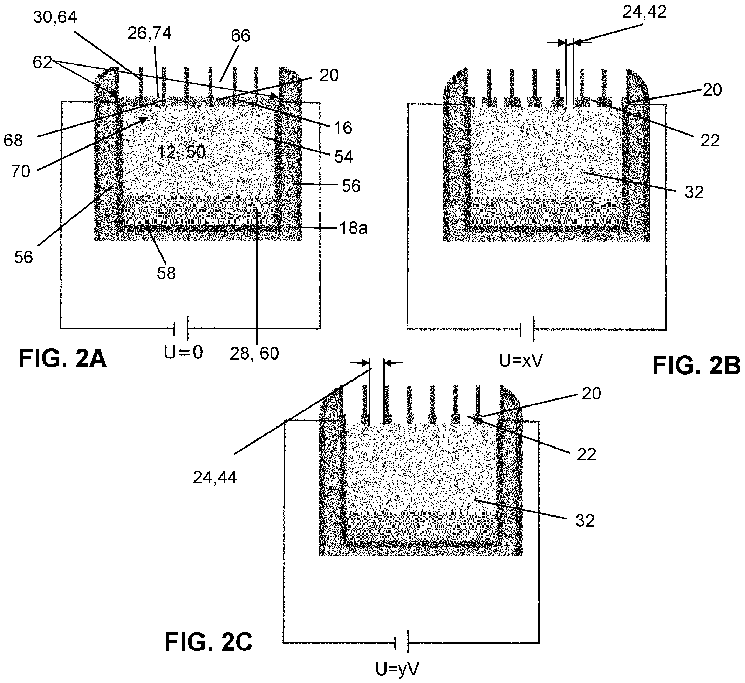

Die Fig. 1 zeigt ein medizinisches Sensorsystem 10 zum Nachweis von einem Merkmal 12 in einem hier nicht gezeigten menschlichen Körper schematisch von oben dargestellt. Das Merkmal 12 stellt einen Analyten 50 in der Form eines nachzuweisenden Proteins dar. Das Sensorsystem 10 weist einen Sensor 14 auf, der in einem Gehäuse 52 angeordnet ist. Wie in der Fig. 2a, die einen Schnitt einlang der Linie II-II in Fig. 1 zeigt, zu sehen ist, weist der Sensor 14 ein Reservoirs 18 bzw. einem Sensorreservoir, auf, das ein Probenvolumen 54 an vier Seiten 56 und einem Boden 58 einschließt, wobei nur zwei Seiten 56 zu sehen sind. In dem Reservoir 18 ist ein Nachweissystem 60 angeordnet, die eine Rezeptorschicht 28 aus Antikörpern gegen das nachzuweisende Protein aufweist bzw. als eine Antikörperschicht auf einem seFET ausgebildet ist.

Zudem weist der Sensor 14 einen Verschluss 16 des Reservoirs 18 des Sensors 14 auf, der an einer sechsten Seite 62 des Reservoirs 18 angeordnet ist bzw. die sechste Seite 62 des Reservoirs 18 bildet. Der Verschluss 16 verschließt zumindest in seinem geschlossenen Zustand das Probenvolumen 54, sodass in diesem Zustand weder eine Struktur noch das Merkmal 12 bzw. der Analyt 50 in das Probevolumen 54 eindringen noch aus diesem austreten kann. Dies ist der Fall vor einer ersten Messung des Sensorsystems 10, aber auch zwischen den verschiedenen Messungen. Der Verschluss 16 ist als steuerbare, organische Membran 20 ausgebildet, die zwischen einem geöffneten und einem geschlossenen Zustand des Reservoirs 18 reversibel veränderbar ist. Hierfür weist die steuerbare, organische Membran 20 mehrere Poren 22 auf, die homogen über die Oberfläche verteilt angeordnet sind und deren Durchmesser 24 reversibel veränderbar sind. In der Fig. 1 sind der Übersichtlichkeit halber nur einige Poren 22 gezeigt. Zudem sind die Poren allen Figuren zur Verdeutlichung des Funktionsprinzips nicht maßstabsgetreu sondern vergrößert gezeigt.

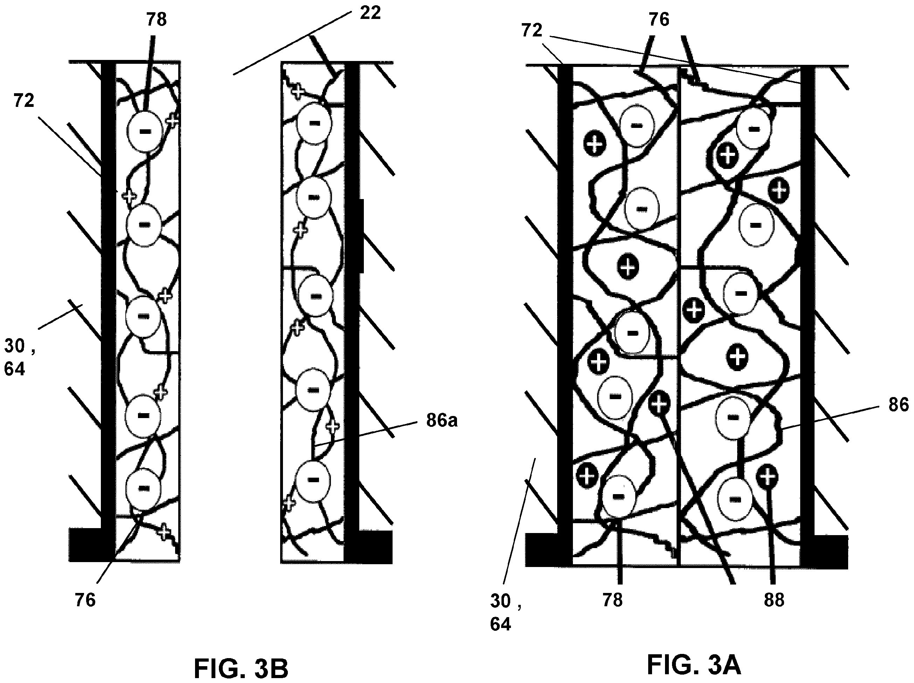

Zudem weist der Verschluss 16 eine Trägerstruktur 30 für die steuerbare, organische Membran 20 auf, die von einem nanoporösen Substrat aus TiO₂ gebildet ist und die somit eine hohe Biokompatibitität aufweist. Die Trägerstruktur 30 ist von sich senkrecht zu dem Boden 58 der Reservoirs 18 und parallel zueinander erstreckenden Nanoröhrchen 64 gebildet. Jedes Nanoröhrchen 64 weist eine für den Analyten 50 permeable Nanopore 66 auf. Für die Messung von Cystatin C ist ein Durchmesser von ca. 10 nm bevorzugt, für die Messung von Glukose ein Durchmesser von ca. 1 nm. Die Innenoberfläche 68 der Nanopore 66 ist an einer zum Boden 58 weisenden Seite 70 (vgl. Fig. 2a) mit einem leitfähigen Material 72 in der Form von Gold durch Sputtern beschichtet. Auf der dem Probenvolumen 54 zugewandten Oberfläche ist die steuerbare, organische Membran 20 angeordnet. Die steuerbare, organische Membran 20 wird aus einer Lösung seiner Komponenten auf der Goldoberfläche der Nanoporen 66 elektropolymerisiert. Zudem ist die steuerbare, organische Membran 20 von einem elektroaktiven Material bzw. Polymer 74 gebildet, das Polypyrrol (PPy) 76 und Dodecylbenzolsulfonsäure (DBS) 78 aufweist (vgl. Fig. 3). Zu einer Steuerung der steuerbare, organische Membran 20 sind Leiterbahnen 80 an die Trägerstruktur 30 auf der Höhe der Goldbeschichtung angebracht und mit einer im Sensor 14 integrierten Steuerung 82 verbunden, wodurch die steuerbare, organische Membran 20 elektrisch steuerbar ist.

Bei einer Herstellung der Trägerstruktur 30 bzw. der Nanoröhrchen 64 kann der Porendurchmesser leicht eingestellt werden, wodurch, abgestimmt auf den einzusetzenden Analyten 50, eine Vielzahl von unterschiedlichen Trägerstrukturen zur Verfügung gestellt werden können, die Grundgerüste für die steuerbare, organische Membran 20 bilden. Eine Schichtdicke der Membran 20 ist hierbei deutlich größer (beispielsweise mehrere 100 µm) als ein Durchmesser der Nanoröhrchen 64.

Durch das elektroaktive Polymer 74 weist die steuerbare, organische Membran 20 ein Material 26 auf, dessen Redoxzustand änderbar ist. Somit können über Kontakte zwischen dem leitfähigen Material 72 und der Steuerung 82, die eine Referenzelektrode 84 aufweist, die Redoxzustände und damit ein Volumen des elektroaktiven Polymers 74 bzw. der steuerbaren, organischen Membran 20 verändert bzw. gesteuert werden. Die Vergrößerung des Volumens führt zum vollständigen Verschluss der Nanoporen 66 der Trägerstruktur 30 und der Poren 22 der steuerbaren, organischen Membran 20, umgekehrt bewirkt die Verkleinerung des Volumens die Öffnung der Nanoporen 66 und der Poren 22 für den Analyten 50. Weil die Reduktion bzw. Oxidation des elektroaktiven Polymers 74 partiell erfolgen kann, ist auch die Öffnung der Nanoporen 66 und der Poren 22 entsprechend partiell und stufenlos regulierbar, was wiederum eine Abstimmung an verschiedene Analyten 50 möglich macht.

Der Sensor 14, die steuerbare, organische Membran 20 und die Trägerstruktur 30 sind so mit dem Gehäuse 52 verbunden, dass ein Stoffaustausch nur über die Porenmembran und nicht über Verbindungsstellen der Komponenten möglich ist

Die Figuren 3a und 3b zeigen eine Pore 22 in einem geschlossenen (Fig. 3a) und in einem offenen Zustand (Fig. 3b). Das elektroaktive Polymer 74 besteht aus einer Matrix 86 quervernetzten positiv geladener Fasern aus Polypyrrol 76. Während der Polymerisation bei der Beschichtung der Goldschicht lagern sich in diese Matrix 86 negativ geladene DBS-Moleküle 78 ein, die aufgrund ihrer Größe nicht aus der Matrix 86 diffundieren können und die negativ geladenen Gegenionen zur positiv geladenen Matrix 86 aus Polypyrrol 76 darstellen. Bei einer vollständigen Reduktion des Polypyrrols 76 wird dieses elektrisch neutral.

Ein Schließen der Poren 22 erfolgt folgendermaßen: Um die negative Ladung der DBS-Moleküle 78 zu kompensieren, werden durch Anlegen einer Spannung von beispielsweise 2 Volt positiv geladene, hydratisierte Natriumionen 88 in die Matrix 86a eingelagert. Dort führen sie zu einer starken (bis zu 30%) lateralen Volumenänderung des elektroaktiven Polymers 74. Diese Änderung des Volumens führt zu einem Verschluss der Poren 22 und verhindert den Eintritt von Strukturen in das Probenvolumen 54. Eine Reversion des Vorganges erfolgt durch Anlegen einer Spannung umgekehrter Polarität, die dann wieder zu einer Volumenreduktion des Polymers 74 führt. Die Reversibilität dieses Vorganges ermöglicht ein wiederholbares Öffnen und Schließen der Poren 22. Ferner ist über den Grad der Reduktion des Volumens des Polymers 74 eine partielle Volumenänderung der steuerbaren, organischen Membran 20 möglich. Die jeweiligen Redoxzustände des elektroaktiven Polymers 74 werden über unterschiedliche angelegte Spannungen erzeugt und können bei einer Abschaltung der Spannung beibehalten werden.

Das Sensorsystem 10 kann auch zu einer quantitativen Bestimmung einer Konzentration des Merkmals 12 bzw. des Analyten 50 verwendet werden, was in den Figuren 2b und 2c gezeigt ist. Hierbei ist derselbe Bereich 32 als der erste Sensor 14 und als ein zweiter Sensor 34 vorgesehen. Zum Nachweis des Merkmals 12 wird hier in einem ersten Schritt ein erster Durchmesser 42 der Pore 22 der steuerbaren, organischen Membran 20 eingestellt und in einem zweiten Schritt ein zweiter Durchmesser 44 der Pore 22 eingestellt, wobei der erste Durchmesser 42 kleiner als der zweite Durchmesser 44 ist. Der erste Schritt stellt eine Referenzmessung dar, bei der so viele Störsignale wie möglichst ermittelt werden sollen. Der erste Durchmesser 42 ist gerade so eingestellt, dass das Merkmal 12 bzw. der Analyt 50 nicht in das Probenvolumen 54 eindringen kann und beträgt beispielsweise ca. 5 nm . Alle kleineren Moleküle, die eine Bestimmung des Analyten 50 beeinträchtigen könnten, können hingegen eindringen. Der zweite Schritt ist eine Analytmessung, hier wird der zweiter Durchmesser 44 gerade so weit auf beispielsweise ca. 10 nm für die Messung von Cystatin C oder auf 1 nm für die Messung von Glukose vergrößert, dass der Analyt 50 nun in den Probenvolumen 54 eindringen kann, um gemessen zu werden. Dies kann durch ein Anlegen unterschiedlicher Spannungen von beispielsweise 1 V bei der Referenzmessung und einer Spannung von 1,5 V bei der Analytmessung erfolgen. Alternativ kann der Durchmesser bei konstant angelegter Spannung (beispielsweise 2 V) durch die Dauer der angelegten Spannung erfolgen. Typische Werte sind 4 Minuten für die Referenzmessung und 5 Minuten bei der Analytmessung. Zum Erhalten eines Endmessergebnisses der Konzentration an Analyt 50 wird nun das Messergebnis der Analytmessung um das Messergebnis der Referenzmessung korrigiert.

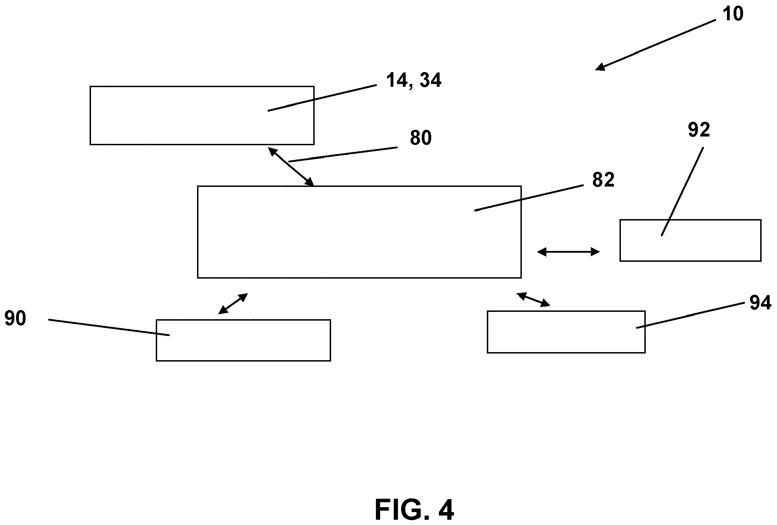

In der Fig. 4 sind schematisch die Bestanteile des Sensorsystems 10 dargestellt. Zusätzlich zu den Sensoren 14, 34 weist das Sensorsystem 10 die Steuerung 82 mit den Leiterbahnen 80 und weiteren nicht gezeigten Elektronikkomponenten, einen Programmspeicher 90, eine Telemetrievorrichtung 92, und eine Stromversorgung 94 auf. Mittels der Telemetrievorrichtung 92 können die vom Sensorsystem erfassten Werte an ein (nicht dargestelltes) externes Gerät übertragen werden. Die Telemetrievorrichtung 92 ist bevorzugt für bidirektionale Kommunikation ausgeführt, so dass das Sensorsystem 10 durch ein externes Gerät gesteuert werden kann. Weiterhin kann das Sensorsystem 10 mittels der Telemetrievorrichtung 92 mit weiteren, implantierten Geräten kommunizieren, um zum Beispiel eine Therapie oder Medikamentenabgabe dieser weiteren implantierten Geräte in Abhängigkeit von den gemessenen Sensorwerten zu steuern.

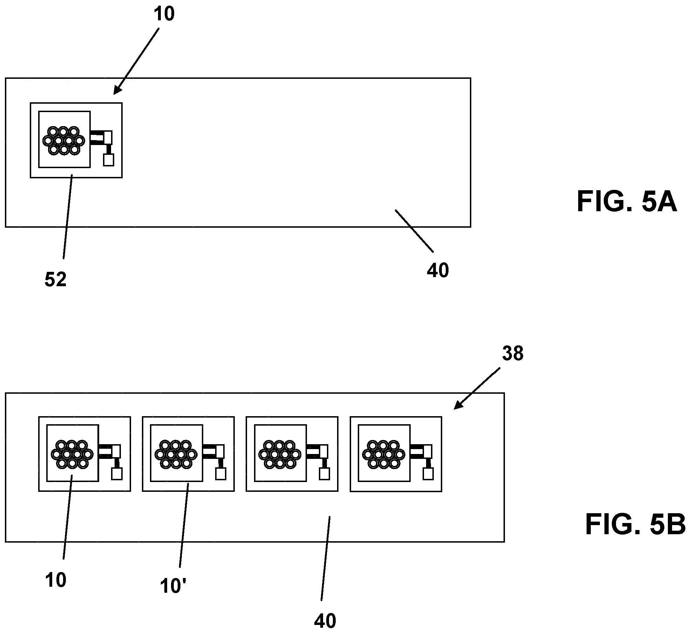

Mehrere Sensorsysteme 10, 10' können in einem medizinischen Sensorarray 38 zusammengefasst werden, wie dies in der Fig. 5b gezeigt ist Hierbei kann nach Verwendung eines ersten Sensorsystems 10 oder nach einem Erreichen dessen Ende einer Lebensdauer des ersten Sensorsystems 10 ein zweites Sensorsystem 10' aktiviert werden.

Zudem ist in den Figuren 5a und 5b zu sehen, dass das Sensorsystem 10 (Fig. 5a) oder das Sensorarray 38 (Fig. 5b) in den menschlichen Körper implantiert werden kann, indem er mit einer nicht im Detail gezeigten Verankerungsvorrichtung an einem medizinischen Implantat 40 befestigt werden kann. Dieses Implantat 40 ist beispielsweise eine Formgedächtnisstruktur, wie ein Stent oder eine Mäanderstruktur zur Implantation in einer Arterie oder Vene (nicht gezeigt). Die Verankerung kann permanent sein oder wieder gelöst werden.

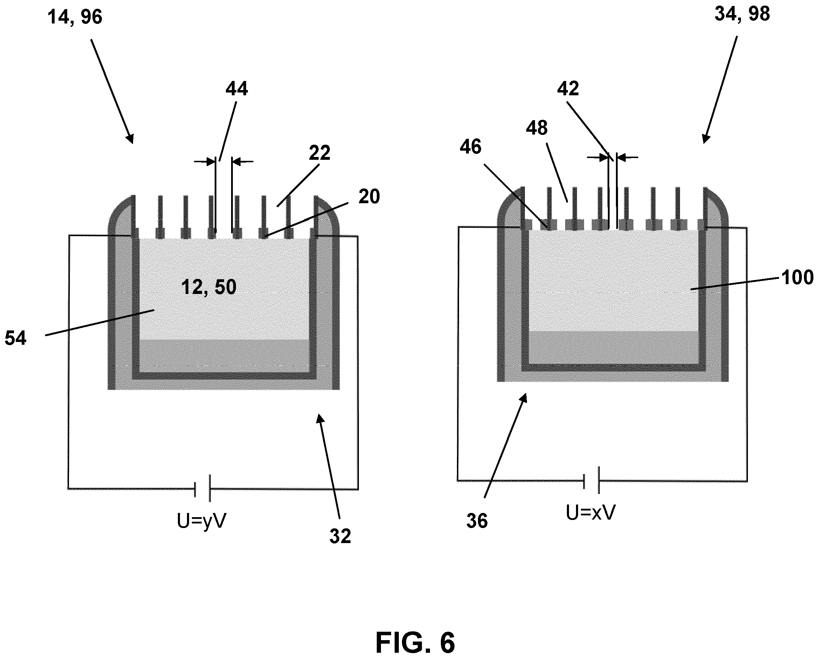

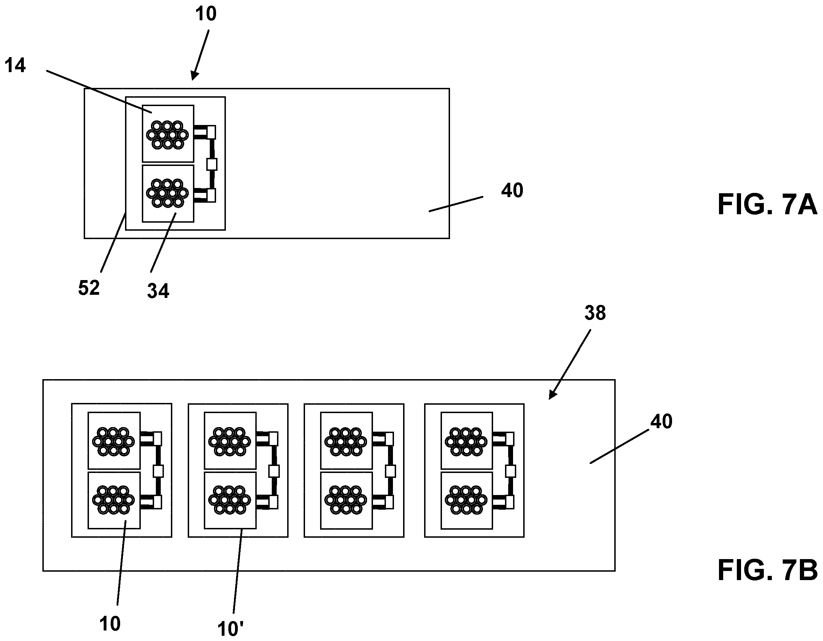

In den Fig. 6, 7a und 7b sind alternative Ausführungsbeispiele des Sensorsystems 10, des Sensorarrays 38 und des Implantats 40 dargestellt. Im Wesentlichen sind gleich bleibende Bauteile, Merkmale und Funktionen grundsätzlich mit den gleichen Bezugszeichen beziffert. Die nachfolgende Beschreibung beschränkt sich im Wesentlichen auf die Unterschiede zu dem Ausführungsbeispiel in den FIG 1 bis 5, wobei bezüglich gleich bleibender Bauteile, Merkmale und Funktionen auf die Beschreibung des Ausführungsbeispiels in den FIG 1 bis 5 verwiesen werden kann.

In der Fig. 6 ist als Schnittdarstellung ein alternatives medizinisches Sensorsystem 10 zum Nachweis von zumindest einem Merkmal 12 in zumindest einem tierischen und/oder menschlichen Körper mit zumindest einem Sensor 14 mit einer Rezeptorschicht 28 als Nachweissystem 60 gezeigt. Das Sensorsystem 10 weist zwei unterschiedliche Bereiche 32, 36 auf, die als der erste Sensor 14 und als ein zweiter Sensor 34 vorgesehen sind. Diese Sensoren 14, 34 sind räumlich voneinander getrennt im gleichen Gehäuse 52 angeordnet (vgl. Fig. 7a). Der erste Sensor 14 stellt einen Messsensor 96 und der zweite Sensor 34 einen Referenzsensor 98 dar. Ferner weist jeder Sensor 14, 34 ein Reservoir 18 auf, das ein Probenvolumen 54 bzw. ein Referenzvolumen 100 einschließt. Jedes Reservoir 18 ist mit einem Verschluss 16 verschlossen. Der Verschluss 16 ist jeweils als elektrisch steuerbare, organische Membran 20, 46 ausgebildet, die ein elektroaktives Polymer 74 aus Polypyrrol und Dodecylbenzolsulfonsäure (nicht im Detail gezeigt) aufweist, wobei das Material 28 des Polymers 74 somit seinen Redoxzustand ändern kann.

Die steuerbaren, organischen Membranen 20, 46 sind jeweils auf eine biokompatible Trägerstruktur 30 aus TiO₂ aufgebracht. Ferner weisen sie Poren 22, 48 auf, deren Durchmesser 24 reversibel änderbar ist, wodurch die steuerbaren, organischen Membranen 20, 46 zwischen einem geöffneten und einem geschlossenen Zustand des Reservoirs 18 reversibel veränderbar sind.

Der Referenzsensor 98 dient zu einer Referenzmessung mittels der ein Messwert einer Analytmessung des Sensors 14 um ein Hintergrundsignal korrigiert werden kann. Hierfür wird zum Nachweis des Merkmals 12 an der steuerbaren, organischen Membran 46 des zweiten Sensors 34 ein erster Durchmesser 42 der Pore 48 der steuerbaren, organischen Membran 46 eingestellt und an der steuerbaren, organischen Membran 20 des ersten Sensors 14 ein zweiter Durchmesser 44 der Pore 22 der steuerbaren, organischen Membran 20 eingestellt, wobei der erste Durchmesser 42 kleiner als der zweite Durchmesser 44 ist. Der erste Durchmesser 42 wird gerade so eingestellt, dass das Merkmal 12 bzw. der Analyt 50 nicht in das Referenzvolumen 100 des Sensors 34 eindringen kann und beträgt beispielsweise ca. 5 nm bei der Messung von Cystatin C und >1nm bei der Messung von Glukose. Alle kleineren Moleküle, die eine Bestimmung des Analyten 50 beeinträchtigen könnten, können hingegen eindringen. Der zweiter Durchmesser 44 der Poren 22 der steuerbaren, organischen Membran 20 des ersten Sensors 14 bzw. des Messsensors 96 wird gerade so weit auf beispielsweise ca. 10 nm für die Messung von Cystatin C oder beispielsweise auf 1 nm bei der Messung von Glukose vergrößert, dass der Analyt 50 in das Probenvolumen 54 eindringen kann, um bestimmt zu werden. Die Sensoren 14, 34 unterscheiden sich in ihrem Aufbau im Messbetrieb also lediglich durch den jeweils eingestellten Durchmesser 42, 44 der Poren 22, 48 der steuerbaren, organischen Membranen 20, 46. Dies kann durch ein Anlegen unterschiedlicher Spannungen von beispielsweise 1 V an der steuerbaren, organischen Membran 20 des ersten Sensors 14 und einer Spannung von 1,5 V an der steuerbaren, organischen Membran 46 des zweiten Sensors 34 erfolgen. Alternativ kann der Durchmesser bei konstant angelegter Spannung (beispielsweise 2 V) durch die Dauer der angelegten Spannung erfolgen. Typische Werte sind 4 Minuten für die Referenzmessung und 5 Minuten bei der Analytmessung.

Die Sensoren 14, 34, die steuerbaren, organischen Membranen 20, 46 und die Trägerstruktur 30 sind so mit dem Gehäuse 52 verbunden, dass ein Stoffaustausch nur über die Porenmembranen und nicht über Verbindungsstellen der Komponenten möglich ist

Grundsätzlich wäre es auch möglich, die steuerbaren, organischen Membranen 20, 46 als eine Membran mit zwei unabhängig voneinander steuerbaren Teilen auszuführen.

Mehrere Sensorsysteme 10, 10' können in einem medizinischen Sensorarray 38 zusammengefasst werden, wie dies in der Fig. 7b gezeigt ist Hierbei kann nach Verwendung eines ersten Sensorsystems 10 oder nach einem Erreichen dessen Ende einer Lebensdauer des ersten Sensorsystems 10 ein zweites Sensorsystem 10' aktiviert werden.

Zudem ist in den Figuren 7a und 7b zu sehen, dass das Sensorsystem 10 (Fig. 7a) oder das Sensorarray 38 (Fig. 7b) in den menschlichen Körper implantiert werden kann, indem er mit einer nicht im Detail gezeigten Verankerungsvorrichtung an einem medizinischen Implantat 40 befestigt werden kann.

**Bezugszeichenliste**

| | | | |
|---|---|---|---|
| 10 | Sensorsystem | 68 | Innenoberfläche |
| 12 | Merkmal | 70 | Seite |
| 14 | Sensor | 72 | Material |
| 16 | Verschluss | 74 | Polymer |
| 18 | Reservoirs | 76 | Polypyrrol |
| 20 | Membran | 78 | Dodecylbenzolsulfonsäure |
| 22 | Pore | 80 | Leiterbahn |
| 24 | Durchmesser | 82 | Steuerung |
| 26 | Material | 84 | Referenzelektrode |
| 28 | Rezeptorschicht | 86 | Matrix |
| 30 | Trägerstruktur | 88 | Natriumion |
| 32 | Bereich | 90 | Programmspeicher |
| 34 | Sensor | 92 | Telemetrievorrichtung |
| 36 | Bereich | 94 | Stromversorgung |
| 38 | Sensorarray | 96 | Messsensor |
| 40 | Implantat | 98 | Referenzsensor |
| 42 | Durchmesser | 100 | Referenzvolumen |
| 44 | Durchmesser | | |
| 46 | Membran | | |
| 48 | Pore | | |
| 50 | Analyt | | |
| 52 | Gehäuse | | |
| 54 | Probenvolumen | | |
| 56 | Seite | | |
| 58 | Boden | | |
| 60 | Nachweissystem | | |
| 62 | Seite | | |
| 64 | Nanoröhrchen | | |
| 66 | Nanopore | | |

## Patentansprüche

1. Medizinisches Sensorsystem (10) zum Nachweis von zumindest einem Analyten (12) in zumindest einem tierischen und/oder menschlichen Körper mit zumindest einem Sensor (14; 34), aufweisend zumindest ein Reservoir (18) mit zumindest einem Nachweissystem (60) für eine Messung des Analyten (12), und zumindest einem Verschluss (16) des Reservoirs (18) des Sensors (14; 34), wobei der Verschluss (16) als steuerbare, organische Membran (20, 46) ausgebildet ist, welche zumindest eine Pore (22, 48) aufweist, deren Durchmesser (24) reversibel veränderbar ist, **dadurch gekennzeichnet, dass** das Sensorsystem derart ausgelegt ist, dass es
entweder:
- in einem ersten Schritt eine Referenzmessung durchführt, wobei ein erster Durchmesser (42) der Pore (22) der steuerbaren, organischen Membran (20) so eingestellt wird, dass der Analyt (12) nicht in ein Probenvolumen (54) des Reservoirs (18) eindringen kann und alle Moleküle, die kleiner als der Analyt (12) sind, in das Probenvolumen (54) eindringen können, wobei bei der Referenzmessung mit dem Nachweissystem (60) Störsignale als ein Messergebnis der Referenzmessung ermittelt werden und
- in einem zweiten Schritt eine Analytmessung durchführt, wobei ein zweiter Durchmesser (44) der Pore (22) der steuerbaren, organischen Membran (20) so eingestellt wird, dass der Analyt (12) in das Probenvolumen (54) eindringen kann, wobei bei der
Analytmessung mit dem Nachweissystem (60) der Analyt (12) als ein Messergebnis der Analytmessung gemessen wird und
- das Messergebnis der Referenzmessung als Hintergrundsignal von dem Messergebnis der Analytmessung abgezogen wird, wodurch das Sensorsystem einen Endmesswert ermittelt,
oder:
- feine Referenzmessung durchführt, wobei ein erster Durchmesser (42) der Pore (48) einer steuerbaren, organischen Membran (46) eines zweiten Sensors (34) so eingestellt wird, dass der Analyt (12) nicht in ein Referenzvolumen (100) eines Reservoirs (18) des zweiten Sensors (34) eindringen kann und alle Moleküle, die kleiner als der Analyt (12) sind, in das Referenzvolumen (100) des Reservoirs (18) des zweiten Sensors (34) eindringen können, wobei bei der Referenzmessung mit dem Nachweissystem (60) Störsignale als ein Messergebnis der Referenzmessung ermittelt werden und
- eine Analytmessung durchführt, wobei an der steuerbaren, organischen Membran (20) des ersten Sensors (14) ein zweiter Durchmesser (44) der Pore (22) der steuerbaren, organischen Membran (20) so eingestellt wird, dass der Analyt (12) in ein Probenvolumen (54) des Reservoirs (18) des ersten Sensors (14) eindringen kann, wobei bei der Messung mit dem Nachweissystem (60) der Analyt (12) als ein Messergebnis der Analytmessung bestimmt wird und
- das Messergebnis der Referenzmessung als Hintergrundsignal von dem Messergebnis der Analytmessung abgezogen wird, wodurch das Sensorsystem einen Endmesswert ermittelt.

2. Medizinisches Sensorsystem nach Anspruch 1, **dadurch gekennzeichnet, dass** die steuerbare, organische Membran (20) zwischen einem geöffneten und einem geschlossenen Zustand des Reservoirs (18) reversibel stufenlos oder in Stufen veränderbar ist.

3. Medizinisches Sensorsystem nach Anspruch 1 oder 2, **dadurch gekennzeichnet, dass** der Durchmesser (24) der zumindest einen Pore (22, 48) der steuerbaren, organischen Membran (20) reversibel stufenlos oder in Stufen veränderbar ist.

4. Medizinisches Sensorsystem nach einem der vorhergehenden Ansprüche, **dadurch gekennzeichnet, dass** die steuerbare, organische Membran (20) zumindest ein Material (26) aufweist, dessen Redoxzustand änderbar ist.

5. Medizinisches Sensorsystem nach einem der vorhergehenden Ansprüche, **dadurch gekennzeichnet, dass** die steuerbare, organische Membran (20) elektrisch steuerbar ist.

6. Medizinisches Sensorsystem nach einem der vorhergehenden Ansprüche, **dadurch gekennzeichnet, dass** zumindest der erste Sensor (14) zumindest eine Rezeptorschicht (28) aufweist.

7. Medizinisches Sensorsystem nach einem der vorhergehenden Ansprüche, **gekennzeichnet durch** eine poröse Trägerstruktur (30) für die steuerbare, organische Membran (20), die zumindest TiO₂ aufweist.

8. Medizinisches Sensorsystem nach einem der vorhergehenden Ansprüche, **dadurch gekennzeichnet, dass** die steuerbare, organische Membran (20) auf eine Trägerstruktur (30) aufgebracht ist, die eine hohe Biokompatibitität aufweist.

9. Medizinisches Sensorsystem nach einem der vorhergehenden Ansprüche, **dadurch gekennzeichnet, dass** derselbe Bereich (32) als ein erster Sensor (14) und als ein zweiter Sensor (34) vorgesehen ist.

10. Medizinisches Sensorsystem nach einem der Ansprüche 1 bis 8, **dadurch gekennzeichnet, dass** zwei unterschiedliche Bereiche (32, 36) als ein erster Sensor (14) und als ein zweiter Sensor (34) vorgesehen sind.

11. Medizinischer Sensorarray (38) mit zumindest zwei Sensorsystemen (10; 10') nach einem der Ansprüche 1 bis 10.

12. Medizinisches Implantat (40) mit zumindest einem medizinischen Sensorsystem (10) nach einem der Ansprüche 1 bis 10 und/oder mit einem medizinischen Sensorarray (38) nach Anspruch 11.

13. Verfahren zum Betreiben eines medizinischen Sensorsystems (10) zum Nachweis von zumindest einem Analyten (12) in zumindest einem tierischen und/oder menschlichen Körper mit zumindest einem Sensor (14; 34), aufweisend zumindest ein Reservoir (18) mit zumindest einem Nachweissystem (60) für eine Analytmessung des Analyten (12), und zumindest einem Verschluss (16) des Reservoirs (18) des Sensors (14; 34), wobei der Verschluss (16) von einer steuerbaren, organischen Membran (20, 46) gebildet wird, die reversibel zwischen einem geöffneten und einem geschlossenen Zustand des Reservoirs (18) verändert wird,
**dadurch gekennzeichnet, dass** entweder:
- in einem ersten Schritt einer Referenzmessung ein erster Durchmesser (42) der Pore (22) der steuerbaren, organischen Membran (20) so eingestellt wird, dass der Analyt (12) nicht in ein Probenvolumen (54) des Reservoirs (18) eindringen kann und alle Moleküle, die kleiner als der Analyt (12) sind, in das Probenvolumen (54) eindringen können, wobei bei der Referenzmessung mit dem Nachweissystem (60) Störsignale als ein Messergebnis der Referenzmessung ermittelt werden und
- in einem zweiten Schritt einer Analytmessung ein zweiter Durchmesser (44) der Pore (22) der steuerbaren, organischen Membran (20) so eingestellt wird, dass der Analyt (12) in das Probenvolumen (54) eindringen kann, wobei bei der Analytmessung mit dem Nachweissystem (60) der Analyt (12) als ein Messergebnis der Analytmessung gemessen wird und
- das Messergebnis der Referenzmessung als Hintergrundsignal von dem Messergebnis der Analytmessung abgezogen wird, wodurch das Sensorsystem einen Endmesswert ermittelt;
oder:
- bei einer Referenzmessung ein erster Durchmesser (42) der Pore (48) einer steuerbaren, organischen Membran (46) eines zweiten Sensors (34) so eingestellt wird, dass der Analyt (12) nicht in ein Referenzvolumen (100) eines Reservoirs (18) des zweiten Sensors (34) eindringen kann und alle Moleküle, die kleiner als der Analyt (12) sind, in das Referenzvolumen (100) des Reservoirs (18) des zweiten Sensors (34) eindringen können, wobei bei der Referenzmessung mit dem Nachweissystem (60) Störsignale als ein Messergebnis der Referenzmessung ermittelt wird und
- bei einer Analytmessung an der steuerbaren, organischen Membran (20) des ersten Sensors (14) ein zweiter Durchmesser (44) der Pore (22) der steuerbaren, organischen Membran (20) so eingestellt wird, dass der Analyt (12) in ein Probenvolumen (54) des Reservoirs (18) des ersten Sensors (14) eindringen kann, wobei bei der Analytmessung mit dem Nachweissystem (60) der Analyt (12) als ein Messergebnis der Analytmessung bestimmt wird und
- das Messergebnis der Referenzmessung als Hintergrundsignal von dem Messergebnis der Analytmessung abgezogen wird, wodurch das Sensorsystem einen Endmesswert ermittelt.

## Claims

1. A medical sensor system (10) for detecting at least one analyte (12) in at least one animal and/or human body, said system comprising at least one sensor (14; 34), having at least one reservoir (18) with at least one detection system (60) for a measurement of the analyte (12), and at least one closure (16) of the reservoir (18) of the sensor (14; 34), wherein the closure (16) is formed as a controllable organic membrane (20, 46), which has at least one pore (22, 48), the diameter of which (24) is reversibly changeable,
**characterised in that**
the sensor system is designed in such a way that it either:
- in a first step performs a reference measurement, wherein a first diameter (42) of the pore (22) of the controllable organic membrane (20) is adjusted such that the analyte (12) cannot infiltrate a sample volume (54) of the reservoir (18) and all molecules that are smaller than the analyte (12) can infiltrate the sample volume (54), wherein, during the reference measurement by means of the detection system (60), interfering signals are determined as a measurement result of the reference measurement, and
- in a second step an analyte measurement is performed, wherein a second diameter (44) of the pore (22) of the controllable organic membrane (20) is adjusted so that the analyte (12) can infiltrate the sample volume (54), wherein, during the analyte measurement by means of the detection system (60), the analyte (12) is measured as a measurement result of the analyte measurement, and
- the measurement result of the reference measurement is removed as background signal from the measurement result of the analyte measurement, whereby the sensor system determines an end measured value,
or:
- a reference measurement is performed, wherein a first diameter (42) of the pore (48) of a controllable organic membrane (46) of a second sensor (34) is adjusted so that the analyte (12) cannot infiltrate a reference volume (100) of a reservoir (18) of the second sensor (34) and all molecules that are smaller than the analyte (12) can infiltrate the reference volume (100) of the reservoir (18) of the second sensor (34), wherein, during the reference measurement by means of the detection system (60), interfering signals are determined as a measurement result of the reference measurement, and
- an analyte measurement is performed, wherein a second diameter (44) of the pore (22) of the controllable organic membrane (20) is adjusted at the controllable organic membrane (20) of the first sensor (14) so that the analyte (12) can infiltrate a sample volume (54) of the reservoir (18) of the first sensor (14), wherein, during the measurement by means of the detection system (60), the analyte (12) is determined as a measurement result of the analyte measurement, and
- the measurement result of the reference measurement is removed as background signal from the measurement result of the analyte measurement, whereby the sensor system determines an end measured value.

2. The medical sensor system according to claim 1, **characterised in that** the controllable organic membrane (20) is reversibly changeable continuously or in steps between an opened and a closed state of the reservoir (18).

3. The medical sensor system according to claim 1 or 2, **characterised in that** the diameter (24) of the at least one pore (22, 48) of the controllable organic membrane (20) is reversibly changeable continuously or in steps.

4. The medical sensor system according to any one of the preceding claims, **characterised in that** the controllable organic membrane (20) comprises at least one material (26), the redox state of which is variable.

5. The medical sensor system according to any one of the preceding claims, **characterised in that** the controllable organic membrane (20) is electrically controllable.

6. The medical sensor system according to any one of the preceding claims, **characterised in that** at least the first sensor (14) has at least one receptor layer (28).

7. The medical sensor system according to any one of the preceding claims, **characterised by** a porous carrier structure (30) for the controllable organic membrane (20), which at least comprises TiO₂.

8. The medical sensor system according to any one of the preceding claims, **characterised in that** the controllable organic membrane (20) is applied to a carrier structure (30) which has a high biocompatibility.

9. The medical sensor system according to any one of the preceding claims, **characterised in that** the same region (32) is provided as a first sensor (14) and as a second sensor (34).

10. The medical sensor system according to any one of claims 1 to 8, **characterised in that** two different regions (32, 36) are provided as a first sensor (14) and as a second sensor (34).

11. A medical sensor array (38) having at least two sensor systems (10; 10') according to any one of claims 1 to 10.

12. A medical implant (40) having at least one medical sensor system (10) according to any one of claims 1 to 10 and/or having a medical sensor array (38) according to claim 11.

13. A method for operating a medical sensor system (10) for detecting at least one analyte (12) in at least one animal and/or human body, comprising at least one sensor (14; 34), having at least one reservoir (18) with at least one detection system (60) for a measurement of the analyte (12), and at least one closure (16) of the reservoir (18) of the sensor (14; 34), wherein the closure (16) is formed by a controllable organic membrane (20, 46), which is reversibly changed between an open and a closed state of the reservoir (18),
**characterised in that**
either:
- in a first step of a reference measurement, a first diameter (42) of the pore (22) of the controllable organic membrane (20) is adjusted such that the analyte (12) cannot infiltrate a sample volume (54) of the reservoir (18) and all molecules that are smaller than the analyte (12) can infiltrate the sample volume (54), wherein, during the reference measurement by means of the detection system (60), interfering signals are determined as a measurement result of the reference measurement, and
- in a second step of an analyte measurement, a second diameter (44) of the pore (22) of the controllable organic membrane (20) is adjusted so that the analyte (12) can infiltrate the sample volume (54), wherein, during the analyte measurement by means of the detection system (60), the analyte (12) is measured as a measurement result of the analyte measurement, and
- the measurement result of the reference measurement is removed as background signal from the measurement result of the analyte measurement, whereby the sensor system determines an end measured value,
or:
- during a reference measurement, a first diameter (42) of the pore (48) of a controllable organic membrane (46) of a second sensor (34) is adjusted so that the analyte (12) cannot infiltrate a reference volume (100) of a reservoir (18) of the second sensor (34) and all molecules that are smaller than the analyte (12) can infiltrate the reference volume (100) of the reservoir (18) of the second sensor (34),
wherein, during the reference measurement by means of the detection system (60), interfering signals are determined as a measurement result of the reference measurement, and
- during an analyte measurement, a second diameter (44) of the pore (22) of the controllable organic membrane (20) is adjusted at the controllable organic membrane (20) of the first sensor (14) so that the analyte (12) can infiltrate a sample volume (54) of the reservoir (18) of the first sensor (14), wherein, during the analyte measurement by means of the detection system (60), the analyte (12) is determined as a measurement result of the analyte measurement, and
- the measurement result of the reference measurement is removed as background signal from the measurement result of the analyte measurement, whereby the sensor system determines an end measured value.

## Revendications

1. Système de capteur (10) médical pour le contrôle d'au moins un analyte (12) dans au moins un corps animal et/ou humain, avec au moins un capteur (14 ; 34) présentant au moins un réservoir (18) avec au moins un système de contrôle (60) pour une mesure de l'analyte (12), et au moins une fermeture (16) du réservoir (18) du capteur (14 ; 34), où la fermeture (16) est conçue sous la forme d'une membrane (20, 46) organique réglable, laquelle présente au moins un pore (22, 48) dont le diamètre (24) peut être modifié de manière réversible,
**caractérisé en ce que**
le système de capteur est conçu de telle manière que,
soit :
- dans une première étape, il effectue une mesure de référence, où un premier diamètre (42) du pore (22) de la membrane (20) organique réglable est réglé de telle façon que l'analyte (12) ne peut pas pénétrer dans un volume échantillon (54) du réservoir (18), et toutes les molécules qui sont plus petites que l'analyte (12) peuvent pénétrer dans le volume échantillon (54), où, lors de la mesure de référence avec le système de contrôle (60), des signaux parasites sont déterminés servant de résultat de la mesure de référence, et
- dans une deuxième étape, il effectue une mesure d'analyte, où un deuxième diamètre (44) du pore (22) de la membrane (20) organique réglable est réglé de telle façon que l'analyte (12) peut pénétrer dans le volume échantillon (54), où, lors de la mesure d'analyte avec le système de contrôle (60), l'analyte (12) est mesuré en tant que résultat de mesure de la mesure d'analyte, et
- le résultat de mesure de la mesure de référence est déduit sous la forme d'un signal de bruit de fond à partir du résultat de mesure de la mesure d'analyte, ce par quoi le système de capteur détermine une valeur finale de mesure,
soit :
- il effectue une mesure de référence, où un premier diamètre (42) du pore (48) d'une membrane (46) organique réglable d'un deuxième capteur (34) est réglé de telle manière que l'analyte (12) ne peut pas pénétrer dans un volume de référence (100) d'un réservoir (18) du deuxième capteur (34) et toutes les molécules qui sont plus petites que l'analyte (12) peuvent pénétrer dans le volume de référence (100) du réservoir (18) du deuxième capteur (34), où, lors de la mesure de référence avec le système de contrôle (60), des signaux parasites sont évalués en tant que résultat de mesure de la mesure de référence, et
- il effectue une mesure d'analyte, où, sur la membrane (20) organique réglable du premier capteur (14), un deuxième diamètre (44) du pore (22) de la membrane (20) organique réglable est réglé de telle façon que l'analyte (12) peut pénétrer dans un volume échantillon (54) du réservoir (18) du premier capteur (14), où, lors de la mesure avec le système de contrôle (60), l'analyte (12) est déterminé sous la forme d'un résultat de mesure de la mesure d'analyte, et
- le résultat de mesure de la mesure de référence est déduit sous forme d'un signal de bruit de fond à partir du résultat de mesure de la mesure d'analyte, ce par quoi le système de capteurs détermine une valeur finale de mesure.

2. Système de capteur médical selon la revendication 1, **caractérisé en ce que** la membrane (20) organique réglable peut être modifiée entre un état ouvert et un état fermé du réservoir (18) de manière réversible sans étape intermédiaire ou par étapes.

3. Système de capteur médical selon la revendication 1 ou 2, **caractérisé en ce que** le diamètre (24) de l'au moins un pore (22, 48) de la membrane (20) organique réglable peut être modifié de manière réversible sans étape intermédiaire ou par étapes.

4. Système de capteur médical selon l'une des revendications précédentes, **caractérisé en ce que** la membrane (20) organique réglable présente au moins un matériau (26) dont l'état rédox est modifiable.

5. Système de capteur médical selon l'une des revendications précédentes, **caractérisé en ce que** la membrane (20) organique réglable peut être commandée électriquement.

6. Système de capteur médical selon l'une des revendications précédentes, **caractérisé en ce qu'**au moins le premier capteur (14) présente au moins une couche de récepteur (28).

7. Système de capteur médical selon l'une des revendications précédentes, **caractérisé par** une structure support (30) poreuse pour la membrane (20) organique réglable qui présente au moins du TiO₂.

8. Système de capteur médical selon l'une des revendications précédentes, **caractérisé en ce que** la membrane (20) organique réglable est rapportée sur une structure support (30) qui présente une biocompatibilité élevée.

9. Système de capteur médical selon l'une des revendications précédentes, **caractérisé en ce que** le même espace (32) est prévu pour servir de premier capteur (14) et de deuxième capteur (34).

10. Système de capteur médical selon l'une des revendications 1 à 8, **caractérisé en ce que** deux espaces (32, 36) différents sont prévus pour servir de premier capteur (14) et de deuxième capteur (34).

11. Ensemble de capteurs (38) médical doté d'au moins deux systèmes de capteurs (10 ; 10') selon l'une des revendications 1 à 10.

12. Implant médical (40) doté d'un système de capteur (10) médical selon l'une des revendications 1 à 10 et/ou doté d'un ensemble de capteurs (38) selon la revendication 11.

13. Procédé d'exploitation d'un système de capteur (10) médical pour le contrôle d'au moins un analyte (12) dans au moins un corps animal et/ou humain avec au moins un capteur (14 ; 34) présentant au moins un réservoir (18) avec au moins un système de contrôle (60) pour une mesure d'analyte de l'analyte (12), et au moins une fermeture (16) du réservoir (18) du capteur (14 ; 34), où la fermeture (16) est conçue sous forme d'une membrane (20, 46) organique réglable, qui peut être modifiée de manière réversible entre un état ouvert et un état fermé du réservoir (18), **caractérisé en ce que**
soit :
- dans une première étape d'une mesure de référence, un premier diamètre (42) du pore (22) de la membrane (20) organique réglable est réglé de telle façon que l'analyte (12) ne peut pas pénétrer dans un volume échantillon (54) du réservoir (18) et toutes les molécules qui sont plus petites que l'analyte (12) peuvent pénétrer dans le volume échantillon (54), où, lors de la mesure de référence avec le système de contrôle (60), des signaux parasites sont déterminés en tant que résultat de la mesure de référence, et
- dans une deuxième étape d'une mesure d'analyte, un deuxième diamètre (44) du pore (22) de la membrane (20) organique réglable est réglé de telle façon que l'analyte (12) peut pénétrer dans le volume échantillon (54), où, lors de la mesure d'analyte avec le système de contrôle (60), l'analyte (12) est mesuré en tant que résultat de mesure de la mesure d'analyte, et
- le résultat de mesure de la mesure de référence est déduit sous la forme d'un signal de bruit de fond à partir du résultat de mesure de la mesure d'analyte, ce par quoi le système de capteur détermine une valeur finale de mesure,
soit :
- lors d'une mesure de référence, un premier diamètre (42) du pore (48) d'une membrane (46) organique réglable d'un deuxième capteur (34) est réglé de telle manière que l'analyte (12) ne peut pas pénétrer dans un volume de référence (100) d'un réservoir (18) du deuxième capteur (34) et toutes les molécules qui sont plus petites que l'analyte (12) peuvent pénétrer dans le volume de référence (100) du réservoir (18) du deuxième capteur (34), où, lors de la mesure de référence avec le système de contrôle (60), des signaux parasites sont évalués en tant que résultat de mesure de la mesure de référence, et
- lors d'une mesure d'analyte, sur la membrane (20) organique réglable du premier capteur (14), un deuxième diamètre (44) du pore (22) de la membrane (20) organique réglable est réglé de telle façon que l'analyte (12) peut pénétrer dans un volume échantillon (54) du réservoir (18) du premier capteur (14), où, lors de la mesure avec le système de contrôle (60), l'analyte (12) est déterminé sous forme d'un résultat de mesure de la mesure d'analyte, et
- le résultat de mesure de la mesure de référence est déduit sous forme d'un signal de bruit de fond à partir du résultat de mesure de la mesure d'analyte, ce par quoi le système de capteurs détermine une valeur finale de mesure.
